(19) Europäisches Patentamt
European Patent Office
Office européen des brevets

(11) Publication number: **0 083 160**
**B1**

(12) **EUROPEAN PATENT SPECIFICATION**

(45) Date of publication of patent specification: **17.08.88**

(51) Int. Cl.$^4$: **B 01 J 29/28,** C 07 C 1/20

(21) Application number: **82306426.6**

(22) Date of filing: **03.12.82**

(54) **Catalyst and process for light olefin production.**

(30) Priority: **30.12.81 US 335982**

(43) Date of publication of application:
**06.07.83 Bulletin 83/27**

(45) Publication of the grant of the patent:
**17.08.88 Bulletin 88/33**

(84) Designated Contracting States:
**BE DE FR GB IT NL**

(56) References cited:
**EP-A-0 023 303**
**FR-A-2 369 997**
**GB-A-1 178 428**
**US-A-3 825 611**
**US-A-4 077 910**
**US-A-4 079 095**
**US-A-4 278 565**

(73) Proprietor: **MOBIL OIL CORPORATION**
**150 East 42nd Street**
**New York New York 10017 (US)**

(72) Inventor: **May-Som Wu, Margaret**
**7 Warrenton Way**
**Belle Mead, NJ 08802 (US)**

(74) Representative: **Cooper, John Anthony et al**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for converting methanol and/or dimethyl ether into light olefins.

A remarkable growth in the production of synthetic fibers, plastics and rubber has taken place in recent decades. Such growth, to a large extent, has been supported and encouraged by an expanding supply of inexpensive petroleum raw materials such as ethylene and propylene. However, increasing demand for these light olefins has, from time to time, led to periods of shortage, due either to a diminished supply of suitable feedstocks or to limited processing capacity. In any event, it is now considered highly desirable to provide efficient means for converting raw materials other than petroleum into light olefins.

One such non-petroleum source of light olefins is coal-derived methanol and dimethyl ether. In this respect, it is known that methanol and dimethyl ether can be catalytically converted into olefin-containing hydrocarbon mixtures by contact under certain conditions with particular types of crystalline zeolite catalyst materials. U.S. Patents 4,025,575 and 4,083,889 for example, both disclose processes whereby methanol and/or dimethyl ether can be converted to an olefin-containing product over a ZSM-5 type (Constraint Index 1—12) zeolite catalyst. ZSM-5, in fact, converts methanol and/or dimethyl ether to hydrocarbons containing a relatively high concentration of light ($C_2$ and $C_3$) olefins with prolonged catalyst lifetime before catalyst regeneration becomes necessary.

It is also known that other types of zeolite catalysts can be used to convert methanol and/or dimethyl ether into olefin-containing hydrocarbon products containing even higher proportions of light olefins that can be realized by methanol/dimethyl ether conversion over ZSM-5. For example, U.S. Patents 4,079,095 and 4,079,096 disclose that zeolites of the erionite-offretite type, and especially ZSM-34, can usefully be employed to promote conversion of methanol and/or dimethyl ether into products comprising a major amount of $C_2$ and $C_3$ light olefins. However, while erionite-offretite type catalysts are extremely selective to light olefins production, such smaller pore zeolites tend to age rapidly in comparison to ZSM-5 when used for methanol/dimethyl ether conversion. There is thus a continuing need to develop new catalyst compositions and systems suitable for selectively converting an organic charge comprising methanol and/or dimethyl ether into light olefin products with both high light olefin selectivity and enhanced catalyst lifetime.

It is also known from U.S. Patent 4,077,910 to activate erionite for hydrocarbon conversion reactions, such as reforming, by subjecting the erionite to the steps of calcination at 480—815°C, contacting with ammonium ions, contacting with cations of a polyvalent metal such as calcium, calcination at 480—815°C, contacting with nickel cations and calcining at 425—760°C.

The present invention resides in a process for converting a feedstock comprising methanol, dimethyl ether or a mixture thereof into a hydrocarbon product rich in ethylene and propylene, which comprises contacting the feedstock at a temperature of from 260°C to 540°C, a pressure from about 0.6 to 2100 kPa and a weight hourly space velocity of the organic reactants of from 0.1 to 30, with a catalyst comprising:

a. a crystalline aluminosilicate zeolite having a crystalline structure having pore windows formed by 8-membered rings or oxygen atoms, and

b. a minor proportion of magnesium oxide, manganese oxide or magnesium oxide and platinum oxide.

The catalyst compositions used in the present process comprise particular types of crystalline aluminosilicate zeolite materials. Such zeolites have crystal structures that provide constrained access to, and egress from, the intracrystalline free space by virtue of having a pore dimension which is usually greater than about 3.6×3.7 Angstroms. Such zeolites also generally have a Constraint Index substantially greater than 12. Zeolite materials of this type have pore windows of about the size provided by 8-membered rings of oxygen atoms. It is to be understood that these rings are those formed by the regular disposition of the $AlO_4$ and $SiO_4$ tetrahedra making up the anionic framework of the crystalline aluminosilicate zeolite, the oxygen atoms themselves being bonded to silicon or aluminum atoms at the centers of the tetrahedra.

These zeolites include zeolite types which may contain some crystalline zeolitic material having pore windows of a size formed by oxygen atom rings containing more than 8 members. For example, a number of natural and synthetic zeolites are known to comprise intergrowths of more than one type of crystalline material. Thus a given zeolite may contain some crystalline material which has pore windows formed by 8-membered rings of oxygen atoms and some material having pore windows formed by 10- or 12-membered rings. The zeolite components of the catalysts of the invention are those which have at least a portion of their total crystalline zeolite material composed of zeolitic material having pore windows formed by 8-membered rings of oxygen atoms.

Zeolites which comprise at least some of the 8-membered ring crystalline zeolite material incude those of the erionite-offretite family such as synthetic and natural erionite, synthetic and natural offretite, Zeolite T, Zeolite W, natural and synthetic chabazite and ZSM-34. Chabazite, erionite and offretite are all more particularly described in Meier and Olson, *Atlas of Zeolite Structure Types,* published in 1978 by the International Zeolite Association and elsewhere. Zeolite T is described in U.S. Patent 2,950,952 and Zeolite W is described in U.S. Patent 3,012,853.

A particularly preferred zeolite material for use in the catalyst compositions applied to the present

invention is ZSM-34. ZSM-34 and its synthesis are more fully described in U.S. Patents 4,116,813, and 4,086,186.

ZSM-34 is a unique crystalline aluminosilicate zeolite, belonging to the erionite-offretite family, having the composition (expressed in terms of molar proportions of constituent oxides) as synthesized, and after drying of:

$$(0.5\text{—}1.3)\ R_2O:(0\text{—}0.15)\ Na_2O:(0.10\text{—}0.50)\ K_2O:Al_2O_3X\ SiO_2$$

where R is the organic nitrogen-containing cation derived from choline $[(CH_3)_3NCH_2CH_2OH]$ and X is 8 to 50, preferably 8 to 30 and still more preferably 8 to 20. This zeolite, unlike other members of the erionite-offretite family, appears to have a tubular morphology and the capability, after calcination at 540°C for at least a period of time sufficient to remove the organic cation, of sorbing at least 9.5 weight percent of n-hexane at ambient temperature and a n-hexane pressure of 2.67 kPa which is higher than that for any other known offretite or erionite. ZSM-34 is characterized by the X-ray powder diffraction pattern set forth in U.S. Patents 4,116,813 and 4,086,186.

All of the above zeolites, as synthesized, may be calcined to remove the organic constituent ($R_2O$) and/or ion exchanged to replace the alkali metal ions with hydrogen ion precursor, for example ammonium, and/or other metal ions, particularly metals from Groups IB, IIA, IIB, IIIB, VIIA, VIII and the rare earth metals with only minor changes in the X-ray characterization and sorption properties. The ion exchanged products are catalytically active zeolites useful in the process of this invention.

For use in carrying out the process of the invention, it may be desirable to incorporate the crystalline aluminosilicate zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, including the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the above materials, the small pore zeolites may be compounded with a porous matrix material such as alumina, silica-alumina, silica-magnesia, silica-zirconia, silica-thoria, silica-beryllia, silica-titania, as well silica-alumina-thoria, silica-alumina-zirconia, silica-alumina-magnesia and silica-magnesia-zirconia. The matrix may be in the form of a cogel. The relative proportions of finely divided zeolite and inorganic oxide gel matrix may vary widely with the zeolite content ranging from 1 to 99 percent by weight and more usually in the range of from 5 to 80 percent by weight of the composite.

As described in more detail below, catalyst compositions comprising a zeolitic material of the 8-membered oxygen ring type as described above can be used to convert a charge comprising methanol and/or dimethyl ether into hydrocarbons. Such catalysts are especially useful in directing this conversion reaction toward light ($C_2$ and $C_3$) olefin production and away from the formation of $C_4^+$ hydrocarbons. It has been discovered that modification of such zeolites with particular types of metal oxides can enhance the lifetime of such zeolite catalysts when they are employed in this selective conversion of methanol and/or dimethyl ether into light olefins.

The zeolite-containing catalysts are modified by incorporating a minor proportion of an oxide of magnesium, an oxide of mangenese or a combination of an oxide of magnesium and an oxide of platinum. Such modified zeolite composites can be prepared by contacting the zeolite composition with one or more compounds or complexes of the metal or metals to be incorporated and thereafter heating the composite to convert the modifying metal into its oxide form. Incorporation can occur by the mechanisms of ion exchange, adsorption and/or impregnation, the latter two phenomena commonly being referred to as "stuffing". It should be emphasized that, while ion exchange can be used to incorporate the modifying metal onto the zeolite, ion exchange alone will generally not provide the requisite amount or form (i.e the oxide form) of incorporated modifying metal onto the zeolite catalyst composition of the invention.

Generally, the zeolite composites can be modified by contact with a solution of a compound of the metal to be incorporated. Such a solution may be formulated from any suitable solvent which is inert with respect to the metal-containing compound and the zeolite composition. Examples of some suitable solvents include water, aromatic and aliphatic hydrocarbons, alcohols, and organic acids (such as acetic acid, formic acid and propionic acid). Other commonly available solvents such as halogenated hydrocarbons, ketones, and ethers may also be useful to dissolve some metal compounds or complexes. Generally, the most useful solvent will be found to be water.

The compound of the modifying metal may also be used without a solvent, i.e., may be used as a neat liquid. Further, the treating compound may also be utilized in the gaseous phase. As such, it can be used alone or in admixture with a gaseous diluent, for example helium or nitrogen, relatively inert to the treating compound and the zeolite.

Treating compounds are those which contain the element magnesium, manganese or platinum, the three catalyst modifiers used in the invention. Magnesium-containing compounds include magnesium

acetate, magnesium nitrate, magnesium benzoate, magnesium propionate, magnesium 2-ethylhexanoate, magnesium carbonate, magnesium formate, magnesium oxylate, magnesium amide, magnesium bromide, magnesium hydride, magnesium lactate, magnesium laurate, magnesium oleate, magnesium palmitate, magnesium salicylate, magnesium stearate and magnesium sulfide.

Manganese-containing compounds include manganese acetate, manganese nitrate, manganese lactate, manganese oxalate, manganese carbonate, manganese citrate, manganese tartarate, manganese bromide, manganese chloride, manganese sulfate and manganese sulfide.

Platinum-containing compounds and complexes include platinum dibromide, platinum carbonyl dichloride, diplatinum dicarbonyl tetrachloride, platinum dichloride, platinum trichloride, platinum hexafluoride, platinum hydroxide, platinum dioxide, platinum pyrophosphate, platimum sulfate, tetraamine platinum chloride and other tetraamine platinum salts.

The amount of metal incorporated onto the zeolite will depend upon several factors. One of these is the reaction time, i.e., the time that the zeolite and the metal-containing source are maintained in contact with each other. With greater reaction times, all other factors being equal, a greater amount of metal is incorporated with the zeolite. Other factors include reaction temperature, concentration of the metal compound in the reaction mixture, the degree to which the zeolite composition has been dried prior to reaction with the metal compound, the conditions of drying of the zeolite composition after reaction with the metal compound, and the amount and type of binder incorporated with the zeolite.

After the metal has been incorporated into the zeolite composite to the extent desired, the metal-containing composite can be heated prior to use. Such heating can be carried out in the presence of oxygen, for example, in air. Although heating may be carried out at a temperature of about 150°C, higher temperatures, for example up to about 500°C, are preferred. Heating is generally carried out for 1 to 5 hours but may be extended to 24 hours or longer. While heating temperatures above about 500°C may be employed, they are generally not necessary. After the metal-containing composite is heated in air at elevated temperatures, it is believed that the modifying metal is actually present at least in part in an oxidized state, such as MgO, MnO and combinations of MgO and PtO.

Generally, the modifying metal oxide is incorporated into the zeolite composite in an amount of from 0.5% to 15% by weight of the zeolite composite, preferably from 1% to 10% by weight of the zeolite composite, calculated on the basis of the elemental metal. When magnesium oxide is the modifying agent, the composite advantageously comprises from 2% to 8% by weight of magnesium. When manganese oxide is the modifying agent, the zeolite composite advantageously comprises from 2% to 8% by weight manganese. When a combination of magnesium oxide and platinum oxide is used as the modifying agent, the zeolite composite advantageously comprises from 0.5% to 8% by weight of total magnesium plus platinum.

The metal oxide-modified zeolite catalysts of the invention are especially useful for the selective conversion of methanol and/or dimethyl ether into hydrocarbons, particularly light ($C_2$—$C_3$) olefins. Processes of this general type are described more fully in U.S. Patents 4,079,095 and 4,079,096.

In accordance with the process of the invention, a chargestock comprising methanol, dimethyl ether, methanol/dimethyl ether mixtures or mixtures of such organic materials with water is contacted in the vapor phase with the modified catalyst materials and under reaction conditions suitable for effecting conversion of methanol and/or dimethyl ether into olefins. Such conditions include an operating temperature of from 260°C to 540°C, preferably 300°C to 450°C; a pressure of from 0.6 to 2100 kPa and preferably 30 to 350 kPa; and a weight hourly space velocity (WHSV) of the organic reactants of from 0.1 to 30, preferably 1 to 10. Carrier gases or diluents, for example, hydrogen, nitrogen, helium, water, carbon monoxide, carbon dioxide, or mixtures of these gases may be injected into the reaction zone.

When water is employed along with the organic feed, the amount of water fed with the organic charge of methanol and/or dimethyl ether may be at least 0.25 moles of water per mole of organic reactants. Preferably, the amount of water added is greater than 0.5 moles of water per mole of organic reactants. The amount of water initially added to the organic charge usually will not exceed about 40 moles per mole of said charge.

The methanol and/or dimethyl ether conversion may be carried out as a batch-type, semi-continuous or continuous operation utilizing a fixed, fluidized or moving bed catalyst system. A preferred process uses a catalyst zone in which the alcohol or ether charge optionally together with added water is passed cocurrently or countercurrently through a fluidized or moving bed of particulate catalyst. The catalyst may then be conducted to a regeneration zone wherein coke is burned from the catalyst in an oxygen-containing atmosphere, for example air, at an elevated temperature, after which the regenerated catalyst can be recycled to the conversion zone for further contact with the methanol- and/or ether-containing feed.

The product stream contains steam and a hydrocarbon mixture of paraffins and olefins, substantially devoid of aromatics. This mixture is particularly rich in light olefins (ethylene and propylene). Generally, a major fraction of the total olefins is ethylene plus propylene with the ethylene content of the product exceeding the propylene content. Thus, the predominant hydrocarbon product constitutes valuable petrochemicals. The steam and hydrocarbon products may be separated from one another by known methods. Preferably, the unconverted methanol and/or dimethyl ether, as well as at least part of the water in the product, is recycled to the reaction zone.

The following Examples illustrate the invention.

Example I

ZSM-34 was prepared by interacting the following solutions:

A. Caustic aluminate

68.89 grams sodium aluminate (20 wt.% Na, 43.1 wt.% $Al_2O_3$, balance $H_2O$)
29.28 grams NaOH (77.5 wt.% $Na_2O$)
26.4 grams KOH (86.4% $K_2O$)
540 grams $H_2O$

B. Silica solution

780 grams colloidal silica sol (30 wt.% $SiO_2$)

C. Choline chloride

228 grams

These components were mixed together in a 2 liter autoclave adding C to A and then adding B followed by 15 minutes continuous mixing. The autoclave was then sealed, pressure-tested and then heated to and held at 150°C for 8 days. The contents were stirred continuously during the 8 day crystallization period.

The autoclave and its contents were then cooled to room temperature, and the crystalline product was filtered and washed. On analysis the product was found to contain:

Na, wt.%: 0.68
K, wt.%: 3.59
$Al_2O_3$, wt.%: 13.5
$SiO_2$, wt.%: 78.5
N, wt.%: 2.5

The resulting ZSM-34 product had the following molar composition:

$$0.54\ R_2O{:}0.11\ Na_2O{:}0.35\ K_2O{:}Al_2O_3{:}9.87\ SiO_2$$

A sample of the calcined alkali ZSM-34 was further processed by contact with a 10 wt.% $NH_4Cl$ solution for 1 hour at about 85°C using 10 ml of solution for each gram of ZSM-34. A total of four contacts were made at these conditions followed by final filtration and water washing essentially free of chloride ions.

The product was dried at 110°C and calcined for 10 hours at 540°C. The residual alkali content as Na was 0.035 wt.% while the residual K content was 1.47 wt.%. This product had a surface area of 517 $m^2/g$ and the following sorption capacities:

Cyclohexane, wt.%: 2.6
n-Hexane, wt.%: 10.0
$H_2O$, wt.%: 18.7

Example II

ZSM-34 prepared in a manner similar to that of Example I was used to convert methanol into hydrocarbons in known manner. The ZSM-34 used in such conversion had a surface area of 475 $m^2/g$ and the following sorption capacities:

Cyclohexane, wt.%: 4.5
n-Hexane, wt.%: 9.9
$H_2O$, wt.%: 16.5

In the conversion procedure, two grams of zeolite (no binder) and four grams of quartz chips, both of 14/20 mesh (1.41/0.84 mm) size, were mixed and packed into a quartz microreactor, equipped with a thermocouple. Several cycles were run, and the catalyst was always calcined at 500°C with air for at least 16 hours before each new cycle. The standard feed contained 37.2% MeOH and 62.8% $H_2O$ (by weight). The methanol/water mixture was fed to the reactor maintained at 370°C and 100 kPa using a weight hourly space velocity (WHSV) of 4.1. The total reactor effluent was analyzed, on line, by a "n-octane on Poracil" column. Methanol conversion was calculated based on hydrocarbon formation only. Selectivities (wt.%) to hydrocarbon product were calculated on "coke free" basis.

The catalyst lifetimes for converting 50% of the methanol for each cycle and the corresponding selectivities to $C_2H_4$, $C_3H_6$ and $C_4H_8$ are summarized in Table I.

TABLE I
Catalyst lifetimes and selectivities to $C_2H_4$, $C_3H_6$ and $C_4H_8$ by HZSM-34 at 50% MeOH conversion

| Cycle | 1 | 2 | 3 | 4 |
|---|---|---|---|---|
| Lifetime for 50% MeOH conversion, (hours) | 2.6 | 2.0 | 1.7 | 1.4 |
| Selectivities (wt.%) at 50% MeOH conversion | | | | |
| $C_2H_4$ | 61 | 56 | 58 | 55 |
| $C_3H_6$ | 25 | 26 | 29 | 28 |
| $C_4H_8$ | 4 | 7 | 7 | 7 |
| Total $C_2^=$—$C_4^=$ | 90 | 89 | 94 | 90 |

The Table I data demonstrate that HZSM-34 provided relatively high selectivity to light olefins for conversion of a methanol/water feed into hydrocarbons. Such data also indicate that catalyst lifetime for methanol conversion over HZSM-34 was relatively short.

Example III

HZSM-34 was modified by incorporating magnesium oxide onto the zeolite in the following manner: two grams of the 14/20 mesh (1.41/0.84 mm) HZSM-34 as prepared in Example I were soaked overnight in an aqueous solution containing 5 grams $Mg(OAc)_2 4H_2O$ and 10 grams distilled water at room temperature. The catalyst pellets were then filtered, dried in air and further dried in an oven at 100°C for 2 hours. The final MgZSM-34, after calcination at 500°C in air for 16 hours, weighed 2.2 g. Catalyst treated in this manner contained about 4.0% by weight magnesium present at least in part in the form of magnesium oxide.

Example IV

The magnesium oxide-modified ZSM-34 prepared as described in Example III was used to promote conversion of methanol into hydrocarbons in the general manner described in Example II. The methanol/water mixture was fed to the reactor at 400°C/100 kPa and a WHSV of 2.1. Five cycles were run. The catalyst lifetimes for converting 50% of the methanol for each cycle and the corresponding selectivities to $C_2H_4$, $C_3H_6$ and $C_4H_8$ are summarized in Table II.

TABLE II
Catalyst lifetimes and selectivities to $C_2H_4$, $C_3H_6$ and $C_4H_8$ by MgZSM-34 at 50% MeOH conversion

| Cycle | 1 | 2 | 3 | 4 | 5 |
|---|---|---|---|---|---|
| Lifetime for 50% MeOH conversion, (hours) | 8.8 | 8.2 | 7.2 | 7.8 | 9.8 |
| Selectivities (wt.%) at 50% MeOH conversion | | | | | |
| $C_2H_4$ | 63 | 60 | 62 | 63 | 64 |
| $C_3H_6$ | 25 | 26 | 26 | 24 | 21 |
| $C_4H_8$ | 4 | 3 | 4 | 4 | 4 |
| Total $C_2^=$—$C_4^=$ | 94 | 89 | 92 | 91 | 89 |

The Table II data in comparison with those of Table I indicate that magnesium modified HZSM-34 improved the catalyst lifetime to 7.2—9.8 hours. The selectivities to $C_2H_4$ and total light olefins were not changed by such catalyst modification.

Example V

HZSM-34 was modified by incorporating manganese oxide onto the zeolite in a manner similar to that described in Example II for the preparation of MgZSM-34. In such a procedure $Mn(OAc)_2 4H_2O$ was used in place of the $Mg(OAc)_2 4H_2O$ employed in Example II. The resulting manganese-treated zeolite contained at least about 2% by weight manganese present at least in part in the form of manganese oxide.

Example VI

The manganese oxide modified ZSM-34 prepared as described in Example V was used to promote conversion of methanol into hydrocarbons in the general manner described in Example II. In such a reaction, the methanol/water mixture was fed to the reactor at 390°C and 100 kPa at a WHSV of 2.1. One cycle was run for which the catalyst lifetime for 50% methanol conversion was 9 hours, and the selectivities to $C_2H_4$, $C_3H_6$ and $C_4H_8$ at 50% methanol conversion were 56%, 30% and 3%, respectively.

Example VII

HZSM-34 was modified by incorporating both magnesium oxide and platinum oxide onto the zeolite in the following manner: two grams HZSM-34 of 14/20 mesh prepared in the general manner described in Example I were gently refluxed for 8 hours in 10 cc aqueous solution containing 0.006 g $Pt(NH_3)_4Cl_2$. The dried zeolite was then soaked in dilute aqueous $Mg(OAc)_2$ (10%) solution for 16 hours at room temperature. The zeolite was then filtered off, dried and calcined in air at 500°C. The zeolite catalyst prepared in this manner contained at least 0.25% by weight magnesium at least in part as magnesium oxide and 0.25% by weight platinum at least in part as platinum oxide.

Example VIII

The magnesium oxide/platinum oxide-modified ZSM-34 prepared as described in Example VII was used to promote conversion of methanol into hydrocarbons in the general manner described in Example II. In such a reaction the methanol/water mixture was fed to the reactor at 370°C/100 kPa using a WHSV of 2.1. Hydrogen was cofed to the reactor at a WHSV for $H_2$ of 0.02. One cycle was run for which the catalyst lifetime for 50% methanol conversion was 7.5 hours, and the selectivities to $C_2H_4$, $C_3H_6$ and $C_4H_8$ were 59%, 27% and 5%, respectively.

Examples VI and VIII show that modification of HZSM-34 with manganese oxide or a combination of magnesium oxide and platinum oxide can improve the catalyst lifetime and maintain the high selectivity to $C_2H_4$ and total light olefin formation for methanol conversion.

Example IX

A synthetic offretite.is prepared by interacting the following

| | | |
|---|---|---|
| Silicate solution: | Q-Brand sodium silicate | 960 g |
| | KOH (88%) | 119.5 g |
| | $H_2O$ | 1050 g |
| Alum solution: | $Al_2(SO_4)_3$ $XH_2O$ | 100.5 g |
| | KCl | 107 g |
| | $H_2O$ | 550 g |
| Tetramethylammonium chloride (50%) | | 128.6 g |

The silicate and alum solutions were mixed in a Waring blender for 10 minutes. The resultant gel was aged at ambient temperature for four hours and then transferred to a 3.8 liter autoclave. TMA Cl was then added to the gel. The mixture was crystallized at 99°C with stirring for about 65 hours. The product mixture was then filtered, washed and dried. The product was TMA offretite, as shown by x-ray diffraction. The crystal size of the product was 0.040—0.2 μm.

The dried zeolite was precalcined at 540°C in flowing $N_2$ for 3 hours, followed by $NH_4NO_3$ exchange to reduce the Na content in the zeolite. The sample was then sized into 14/20 mesh (1.41/0.84 mm) and air calcined at 540°C for 3 hours. The final product was analyzed and found to contain 0.02% weight Na and 2.4% weight K.

Two grams of the synthetic offretite so synthesized were then soaked in an aqueous solution containing 5 grams of $Mg(OAc)_2 \cdot 4H_2O$ and 10 grams of distilled water at room temperature overnight. The catalyst pellets were then filtered, dried in air and further dried in an oven at 100°C for 2 hours. The final Mg offretite after calcination in air at 500°C for 16 hours, contained at least 2% by weight of magnesium present at least in part in the form of magnesium oxide. This modified offretite zeolite was an effective catalyst for the selective conversion of methanol and of dimethyl ether into light olefins with improved catalyst lifetime in comparison to similar offretite material containing no magnesium oxide.

Example X

Synthetic erionite was prepared by interacting the following

A. Sodium aluminate solution

98.2 g $NaAlO_2$ (41.8 wt.% $Al_2O_3$, 33.1 wt.% $Na_2O$)
1680 ml $H_2O$
208 g NaOH 97 wt.%
42.4 g KOH 85.5 wt.%

B. Colloidal silica
234 g colloidal silica (30 wt.% $SiO_2$)

C. Benzyltrimethyl ammonium chloride
142 g 60 wt.% solution

These were mixed, adding C to A and then adding B. After mixing for 15 minutes the slurry was transferred to two polypropylene jars and reacted in a 100°C bath for 68 days.

The adsorption capacities of a sample of the resulting crystalline synthetic erionite after calcination for 10 hours at 540°C were:

Cyclohexane, wt.%: 1.0
n-Hexane, wt.%: 8.4
$H_2O$: 16.6
$m^2/g$ is 447

The synthetic erionite prepared above was calcined for 10 hours at 540°C and then contacted 4 times with 113 ml portions of 0.5 N $NH_4Cl$ solution at 88—90°C. The exchanged zeolite was then water washed essentially free of chloride ions, dried at 110°C, pelleted and sized 14—25 mesh and then calcined for 10 hours at 540°C. The residual sodium content was 0.18 weight percent.

The synthetic erionite prepared above was further modified to incorporate magnesium oxide onto the zeolite. Two grams of the synthetic erionite pellets were soaked in an aqueous solution containing 5 grams $Mg(OAc) \cdot 4H_2O$ and 10 grams distilled water at room temperature overnight. The catalyst pellets were then filtered, dried in air and further dried in an oven at 100°C for 2 hours. The final Mg-erionite, after calcination at 500°C in air for 16 hours, contained at least 2% by weight magnesium present at least in part as magnesium oxide. Such a modified erionite zeolite was an effective catalyst for the selective conversion of methanol or dimethyl ether into light olefins with improved catalyst lifetime in comparison to similar erionite material containing no magnesium oxide.

Example XI

Zeolite T was prepared in accordance with Example 1 of U.S. Patent 2,950,952. The sorption capacities of a sample of the resulting Zeolite T calcined at 540°C were as follows:

Cyclohexane, wt.%: 0.9
n-Hexane, wt.%: 2.0
$H_2O$, wt.%: 12.6

The alkali zeolite was subsequently processed by calcining in air for 10 hours at 540°C and then exchanged for 2 to 4 hours by contact with 5 M $NH_4Cl$ at 82°C using 6 ml of solution per gram of zeolite. This treatment was followed by water washing essentially free of Cl ions, drying and recalcining for 10 hours at 540°C. The base exchange step was repeated to reduce the residual alkali content to a low level. The water-washed, exchanged zeolite was air dried at 110°C, pelleted and sized 14—25 mesh (1.41/0.71 mm) and recalcined for 10 hours at 540°C.

An analysis of the final zeolite showed the following composition:

Na, wt.%: 0.075
K, wt.%: 1.65
$Al_2O_3$, wt.%: 18.7
$SiO_2$, wt.%: 78.8
Molar ratio $SiO_2/Al_2O_3$ 7.2

The sorption capacities were as follows:

Cyclohexane, wt.%: 0.6
n-Hexane, wt.%: 5.7
$H_2O$, wt.%: 13.1
Surface area was 199 $m^2/g$

Zeolite T prepared above was modified to incorporate magnesium oxide onto the zeolite. Two grams of the Zeolite T pellets were soaked in an aqueous solution containing 5 g of $Mg(OAc)_2 4H_2O$ in 10 g of distilled water for 16 hours at room temperature. The zeolite was then filtered, dried and calcined in air at 500°C. The zeolite prepared in this manner contained at least 2% by weight magnesium as magnesium oxide. Such a modified Zeolite T zeolite was an effective catalyst for the selective conversion of methanol or dimethyl ether into light olefins with improved catalyst lifetime in comparison to similar Zeolite T material containing no modifying oxide.

**Claims**

1. A process for converting a feedstock comprising methanol, dimethyl ether or a mixture thereof into a hydrocarbon product rich in ethylene and propylene, which comprises contacting the feedstock at a temperature of from 260°C to 540°C, a pressure from about 0.6 to 2100 kPa and a weight hourly space velocity of the organic reactants of from 0.1 to 30, with a catalyst comprising:

a. a crystalline aluminosilicate zeolite having a crystalline structure having pore windows formed by 8-membered rings or oxygen atoms, and

b. a minor proportion of magnesium oxide, manganese oxide or magnesium oxide and platinum oxide.

2. A process according to claim 1, wherein the zeolite is erionite, offretite, chabazite, Zeolite T or Zeolite W.

3. A process according to claim 1, wherein the zeolite is ZSM-34.

4. A process according to any one of claims 1 to 3, wherein the zeolite is combined with a binder.

5. A process according to any one of claims 1 to 4, wherein b. is magnesium oxide in an amount corresponding to from 1% to 10% by weight of magnesium.

6. A process according to any one of claims 1 to 4, wherein b. is manganese oxide in an amount corresponding to from 1% to 10% by weight of manganese.

7. A process according to any one of claims 1 to 4, wherein b. is magnesium oxide and platinum oxide in amounts corresponding to from 0.5% to 8% by weight magnesium plus platinum.

8. A process according to any preceding claim, carried out at a temperature from 300°C to 450°C, a pressure of from 30 to 350 kPa and a weight hourly space velocity of from 1 to 10.

9. A process according to any preceding claim, wherein the feedstock also comprises water in an amount of at least 0.25 mole of water per mole of organic reactants.

**Patentansprüche**

1. Verfahren zur Umwandlung eines Ausgangsmaterials, das Methanol, Dimethylether oder eine Mischung davon umfaßt, in ein an Ethylen und Propylen reiches Kohlenwasserstoffprodukt, bei dem dieses Ausgangsmaterial bei einer Temperatur von 260 bis 540°C, einem Druck von etwa 0,6 bis 2100 kPa und einer stündlichen Gewichts-Raum-Geschwindigkeit der organischen Reaktanten von 0,1 bis 30 mit einem Katalysator in Kontakt gebracht wird, welcher umfaßt:

a) einen kristallinen Aluminosilicatzeolith mit einer Kristallstruktur, die durch 8-gliedrige Ringe oder Sauerstoffatome gebildete Porenfenster aufweist, und

b) einen geringen Anteil an Magnesiumoxid, Manganoxid oder Magnesiumoxid und Platinoxid.

2. Verfahren nach Anspruch 1, worin der Zeolith Erionit, Offretit, Chabazit, Zeolith T oder Zeolith W ist.

3. Verfahren nach Anspruch 1, worin der Zeolith ZSM-34 ist.

4. Verfahren nach einem der Ansprüche 1 bis 3, worin der Zeolith mit einem Bindemittel kombiniert ist.

5. Verfahren nach einem der Ansprüche 1 bis 4, worin b) Magnesiumoxid in einer Menge ist, die von 1 bis 10 Gew.-% Magnesium entspricht.

6. Verfahren nach einem der Ansprüche 1 bis 4, worin b) Manganoxid in einer Menge ist, die von 1 bis 10 Gew.-% Mangan entspricht.

7. Verfahren nach einem der Ansprüche 1 bis 4, worin b) Magnesiumoxid und Platinoxid in Mengen ist, die von 0,5 bis 8 Gew.-% Magnesium plus Platin entspricht.

8. Verfahren nach einem der vorstehenden Ansprüche, das bei einer Temperatur von 300 bis 450°C, einem Druck von 30 bis 350 kPa und einer stündlichen Gewichts-Raum-Geschwindigkeit von 1 bis 10 durchgeführt wird.

9. Verfahren nach einem der vorstehenden Ansprüche, worin das Ausgangsmaterial ebenfalls Wasser in einer Menge von mindestens 0,25 Mol Wasser pro Mol der organischen Reaktanten umfaßt.

**Revendications**

1. Un procédé de conversion d'une charge comprenant du méthanol, du diméthyléther ou leurs mélanges, en un hydrocarbure riche en éthylène et en propylène qui consiste à mettre la charge, à une température de 260 à 540°C, sous une pression d'environ 0,6 à 2 100 kPa et à une vitesse spatiale horaire pondérale des réactifs organiques comprise entre 0,1 et 30, au contact d'un catalyseur comprenant:

a) une zéolite à base d'aluminosilicate cristalline dont la structure cristalline renferme des fenêtres de pores formées de cycle à huit éléments d'atomes d'oxygène; et

b) une proportion mineure d'oxyde de magnésium, d'oxyde de manganèse ou d'oxyde de magnésium et d'oxyde de platine.

2. Un procédé selon la revendication 1, dans lequel la zéolite est l'érionite, l'offretite, la chabazite, la zéolite T ou la zéolite W.

3. Un procédé selon la revendication 1, dans lequel la zéolite est la ZSM-34.

4. Un procédé selon l'une quelconque des revendications 1 à 3, dans lequel la zéolite est associée à un liant.

5. Un procédé selon l'une quelconque des revendications 1 à 4, dans lequel b) représente de l'oxyde de magnésium en quantité correspondant à 1 à 10% en poids de magnésium.

6. Un procédé selon l'une quelconque des revendications 1 à 4, dans lequel b) représente de l'oxyde de manganèse en quantité correspondant à 1 à 10% en poids de manganèse.

7. Un procédé selon l'une quelconque des revendications 1 à 4, dans lequel b) représente de l'oxyde de

magnésium et de l'oxyde de platine en quantités correspondant à un total magnésium+platine de 0,5 à 8% en poids.

8. Un procédé selon l'une quelconque des revendications précédentes, mis en oeuvre à une température de 300 à 450°C, sous une pression de 30 à 350 kPa et à une vitesse spatiale horaire pondérale de 1 à 10.

9. Un procédé selon l'une quelconque des revendications précédentes, dans lequel la charge comprend également de l'eau à raison d'au moins 0,25 mole d'eau par mole de réactifs organiques.